(19) Europäisches Patentamt
European Patent Office
Office européen des brevets

(11) **EP 3 558 400 B1**

(12) **EUROPÄISCHE PATENTSCHRIFT**

(45) Veröffentlichungstag und Bekanntmachung des Hinweises auf die Patenterteilung:
**10.11.2021  Patentblatt 2021/45**

(51) Int Cl.:
*A61L 15/60* *(2006.01)*     *A61L 15/26* *(2006.01)*
*A61L 26/00* *(2006.01)*

(21) Anmeldenummer: **17825217.7**

(22) Anmeldetag: **21.12.2017**

(86) Internationale Anmeldenummer:
**PCT/EP2017/084043**

(87) Internationale Veröffentlichungsnummer:
**WO 2018/115257 (28.06.2018 Gazette 2018/26)**

(54) **WASSERHALTIGE HYDROGELE ZUR WUNDVERSORGUNG**

WATER-CONTAINING HYDROGELS FOR DRESSING WOUNDS

HYDROGELS AQUEUX POUR LE TRAITEMENT DE PLAIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2016  DE 102016125534**

(43) Veröffentlichungstag der Anmeldung:
**30.10.2019  Patentblatt 2019/44**

(73) Patentinhaber: **Paul Hartmann AG**
**89522 Heidenheim (DE)**

(72) Erfinder:
• **KETTEL, Markus**
**89520 Heidenheim an der Brenz (DE)**
• **RICHTER, Hanno**
**91096 Möhrendorf (DE)**

(56) Entgegenhaltungen:
**EP-A1- 2 338 529     EP-A1- 2 767 293**
**EP-A2- 0 426 422**

**Beschreibung**

Der Gegenstand der vorliegenden Erfindung umfasst wasserhaltige Hydrogele zur Behandlung von Wunden.

Die Heilung von Hautwunden beruht auf der Fähigkeit der Haut, Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur drei wesentliche Heilungsphasen einer Wunde, insbesondere bei Wunden mit Gewebeverlust, beschrieben. Hierzu gehört die Entzündungs- (inflammatorische) oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase). Es hat sich gezeigt, dass eine Abheilung der Wunde durch eine feuchte Wundbehandlung besonders gefördert wird. Im Rahmen einer Wundbehandlung können unter anderem Wundauflagen aus verschiedenen Materialien eingesetzt werden.

Es ist bekannt, dass es bei der Wundheilung zu Störungen kommen kann. Im Falle einer gestörten Wundheilung können Nekrosen und pathologische Mikroorganismen auf den physiologischen Metabolismus während des Wundheilungsgeschehens negativ einwirken. Dies kann häufig zu lokaler Hypoxie führen, was dann in einem weiteren Abbau des umliegenden Gewebes resultieren kann. Dieser Abbau des umliegenden Gewebes kann wiederum die Wundheilung zusätzlich behindern, wobei chronische Wunden entstehen können. Als chronische Wunden werden im Rahmen dieser Erfindung Wunden bezeichnet, die nicht in einem erwarteten Zeitraum von 4 bis 6 Wochen verheilen.

Weiterhin können bei der nicht optimalen Behandlung von Wunden Narben zurückbleiben. Im günstigen Fall wird das betroffene Gewebe durch die Narben nur aus kosmetischer Sicht aber nicht in seiner Wirkungsweise beeinträchtigt. Andernfalls ist es aber auch möglich, dass das vernarbte Gewebe funktionelle Eigenschaften, wie seine Elastizität und Empfindlichkeit, einbüßt.

Aus Wunden tritt ein Exsudat aus, das eine komplexe Zusammensetzung aufweist. Es enthält dabei sowohl wachstumsfördernde Substanzen als auch den Gewebeaufbau erschwerender Substanzen, deren gegensätzliche Wirkungen entscheidenden Einfluss auf den Wundheilungsverlauf haben.

Wasserhaltige Hydrogele werden als Bestandteil von Wundverbänden erfolgreich in der feuchten Behandlung von Wunden eingesetzt. Sie sind in der Lage, aus der Wunde austretendes Wundexsudat in die Gelmatrix aufzunehmen und Feuchtigkeit aus der Gelmatrix abzugeben, so dass der Wunde ein die Wundheilung förderndes Milieu zur Verfügung gestellt wird. Häufig stellen sie die Wundkontaktschichten von Wundverbänden dar. Im Laufe der Zeit, in der die Wundauflagen auf der zu behandelnden Wunde aufliegen, werden dabei die Absorptionskapazität und das Feuchtigkeitsabgabevermögen erschöpft, so dass die ein wasserhaltiges Hydrogel enthaltenden Wundverbände bis zur vollständigen Wundheilung mehrfach gewechselt werden müssen. Ein Verbandwechsel stellt dabei für den Patienten eine kritische Situation dar, weil mit dem Verband am Verband anhaftende Bestandteile des Wundgewebes oder der empfindlichen, die Wunde umgebenden Haut mitgerissen werden können. Um den Verbandwechsel so wenig traumatisch wie möglich gestalten zu können, werden besondere Anforderungen an die Wundkontaktschicht gestellt. So darf das Anhaftungsvermögen des Hydrogels an Wundgewebe und/oder Haut nicht zu groß sein, obwohl ein initiales Anhaften des Hydrogels beim Auflegen der Wundauflage zur besseren Fixierbarkeit durchaus gewünscht ist.

Weiterhin ist es zur Aufrechterhaltung eines wundheilungsfördernden Milieus wünschenswert, eine zellkompatible Hydrogelzusammensetzung zur Verfügung zu stellen. Vorteilhaft ist eine Zusammensetzung, die in der Lage ist, schädliche Faktoren des Wundexsudats zu binden und so von dem Wundgewebe zu entfernen, sowie wundheilungsfördernde Komponenten des Wundexsudats aufzukonzentrieren und so dem Wundgewebe in höherer Konzentration zur Verfügung zu stellen.

Schließlich sollen Wundauflagen für den Patienten angenehm zu tragen sein und möglichst wenig die Bewegungsfreiheit der betroffenen Körperteile einschränken, um die Therapietreue des Patienten nicht nachteilig zu beeinflussen.

EP630629 offenbart wasserhaltige Hydrogelmatrices umfassend ein Polyurethan-Polyharnstoff-Copolymer und Propylenglykol zur Wundbehandlung.

WO2010/000450 und WO2010/000451 offenbaren Schaumstoff-haltige Wundauflagen mit einer wasserhaltigen Hydrogelmatrix umfassend ein Polyurethan-Polyharnstoff-Copolymer.

EP2338528 und EP2338529 offenbaren wasserhaltige Hydrogelmatrices umfassend ein Polyharnstoff-Polyurethan-Copolymer und Propylenglykol mit verbesserten Absorptions- bzw. Haftungseigenschaften.

Es hat sich jedoch heraus gestellt, dass sowohl die Wundheilung in dem oben stehenden Verfahren noch verbesserungsfähig ist. Insbesondere der Bestandteil Propylenglykol wurde als nachteilig identifiziert. Auch können die bekannten Wundauflagen zu Hautirritationen führen und sind im Hinblick auf die Therapietreue der Patienten noch optimierbar.

Es besteht ein ständiges Interesse daran, Mittel bereitzustellen, die die genannten Anforderungen besser erfüllen. Der Gegenstand des Anspruchs 1 löst diese Aufgabe.

Ziel der vorliegenden Erfindung ist es, die Nachteile aus dem Stand der Technik zu überwinden.

Insbesondere liegt die Aufgabe zugrunde, die Verwendung von Substanzen in der Behandlung von Wunden zur Verfügung zu stellen, welche zu einer verbesserten Wundheilung führen. Die Substanzen beziehungsweise die diese Substanzen enthaltenden Wundversorgungsprodukte sollen vom Patienten als angenehm empfunden werden, eine gute Verträglichkeit für Wundgewebe und Haut aufweisen und zu einer vorteilhaften Therapietreue führen.

Somit ist es eine Aufgabe der vorliegenden Erfindung, ein verbessertes Wundversorgungsprodukt bereitzustellen, das den pathologischen Zustand einer Wunde derart beeinflusst, dass ein schneller Wundheilungsverlauf stattfinden kann. Dieses Wundversorgungsprodukt soll bedarfsgerecht in allen Phasen der Wundheilung angebracht werden können, um den oben genannten Effekt zu gewährleisten.

Im Sinne dieser Erfindung liegt üblicherweise eine Wunde vor, wenn an einer äußeren oder inneren Körperoberfläche der Gewebezusammenhang getrennt wurde.

Es gibt unterschiedliche Arten von Wunden. So wird unter einer primär heilenden Wunde eine Wunde mit nicht klaffenden Wundrändern verstanden, welche sich durch komplikationslose Heilung ohne Infektion auszeichnet. Diese Wunden kommen häufig in gut durchbluteten Körperteilen vor. Die nicht klaffenden und damit eng aneinander liegenden Wundränder können beispielsweise durch einen (chirurgischen) Schnitt verursacht worden sein. Erfolgt keine weitere Behandlung schließt sich die Wunde komplikationslos.

Weitere Wunden sind die sekundär heilenden Wunden. Unter einer sekundär heilenden Wunde wird eine Wunde verstanden, wenn a) ein Verlust des Gewebes vorliegt und/oder b) eine Verkeimung eingetreten ist, welche die primäre Heilung verhindert. Den Verlust an Gewebe kann der Organismus durch neu zu bildendes Gewebe und Überhäutung ausgleichen. Dies führt im Rahmen der sekundären Wundheilung über die Bildung eines Granulationsgewebes bis zum narbigen Ersatz der Gewebslücke.

In einer bevorzugten Ausführungsform der vorliegenden Erfindung ist die sekundär heilende Wunde eine mechanische Wunde, eine thermische Wunde, eine durch chemische Substanzen oder durch Strahlung verursachte Wunde.

Durch äußere Gewalteinwirkung können mechanische Wunden entstehen. Hierzu zählen beispielsweise Schnitt-, Stich-, Platz-, Quetsch-, Schürf-, Kratz-, Biss- und Schusswunden.

Thermische Wunden werden im Wesentlichen durch starke Wärme oder Kälteeinwirkung verursacht. Hierzu zählen beispielsweise Verbrennungen, Verbrühungen, Erfrierungen sowie durch elektrischen Strom verursachte Verletzungen.

Unter chemischen Wunden werden Verätzungen verstanden. Diese können beispielsweise durch saure, alkalische, oxidierende und/oder reduzierende Substanzen verursacht werden.

Durch Strahlung verursachte Wunden werden auch als aktinische Wunden bezeichnet. Diese werden beispielsweise durch ionisierende Strahlung ausgelöst und können ein ähnliches Erscheinungsbild wie Verbrennungswunden aufweisen.

Chronische Wunden können definiert werden als Wunden, deren Heilungsverlauf in einem oder allen Stadien der Wundheilung von der normalen Wundheilung abweichen. So kann aus akuten, normal heilenden Wunden z.B. durch eine Wundinfektion eine chronische Wunde entstehen, die durch eine verzögerte Heilungsgeschwindigkeit gekennzeichnet ist. Der Übergang von einer akuten zu einer chronischen Wunde kann dabei in jedem Stadium der Wundheilung erfolgen. Klinisch werden chronische Wunden definiert als Wunden, deren Heilung mehr als 6-8 Wochen benötigen, wobei diese Definition nicht alle Krankheitsbilder korrekt abdeckt. Es handelt sich bei chronischen Wunden mehr um eine Diagnose, die sich auf die klinische Erfahrung des medizinischen Personals stützt.

Chronische Wunden entstehen insbesondere aufgrund einer mechanischen Belastung (Dekubitus, Druck-Ulzera, Druckgeschwür), einer venösen Insuffizienz (Ulcus cruris venosum, venöse Ulzera), einer arteriosklerotischen Gefäßveränderung (Ulcus cruris arteriosum, arterielle Ulzera), einer neuropathischen Veränderungen (diabetisches Fußsyndrom, neuropathische Ulzera), aber auch in Folge von Autoimmunerkrankungen, von Tumoren (exulzierende Tumore) oder Strahlenschäden bei der Tumortherapie.

Der Dekubitus ist definiert als durch äußere (längerfristige) Druckeinwirkung mit Kompression von Gefäßen und lokaler Ischämie hervorgerufene trophische Störung von Geweben (v. a. Haut und Unterhautgewebe) mit Nekrose, Mazeration, evtl. Infektion. Dekubitalulcera entstehen vor allem bei Bettlägerigkeit, insbesondere an Körperstellen, an denen die Haut dem Knochen unmittelbar anliegt, aber auch z.B. unter schlecht sitzenden Prothesen und zu engen Gipsverbänden.

Der Dekubitus wird in folgende Stadien eingeteilt. Hierbei sind als chronische Wunden insbesondere Dekubitus der Stufe II, Stufe III und Stufe IV bekannt:

Dekubitus - Stufe I: Hierbei handelt es sich um eine persistierende, umschriebene Hautrötung, die auch bei Entlastung bestehen bleibt. Die Rötung ist scharf umgrenzt und kann verhärten oder überwärmt sein. Die Haut ist noch intakt.

Dekubitus - Stufe II: In dieser Phase kommt es zu Blasenbildung und Hautabschürfung und damit zu Teilverlust der Haut. Die Epidermis bis hin zu Anteilen der Dermis ist geschädigt. In dieser Phase liegt eine oberflächige Wunde oder flaches Geschwür vor.

Dekubitus - Stufe III: In diesem fortgeschrittenen Stadium ist bereits ein Verlust aller Hautschichten zu beobachten. Darüber hinaus sind eine Schädigung der subkutanen Gewebe und eventuell Nekrosen zu beobachten, die bis auf das darunter liegende Muskelgewebe reichen können. Erfahrungsgemäß muss es erst zu einer Abgrenzung des nekrotischen Gewebes kommen bis das ganze Ausmaß des Gewebeschadens erkennbar wird. Der Dekubitus III zeigt sich klinisch als offenes, tiefes Geschwür.

Dekubitus - Stufe IV: In diesem äußerst kritischen Stadium ist ein Verlust aller Hautschichten mit ausgedehnter Zerstörung, Gewebsnekrose oder Schädigung von Muskeln, Knochen oder unterstützenden Strukturen (Sehnen, Gelenkkapseln) zu verzeichnen. Der Dekubitus IV zeigt sich klinisch als großflächiges, offenes und tiefes Geschwür.

Die inflammatorische Phase tritt üblicherweise direkt nach dem Trauma auf und dauert in etwa drei Tage. Sie ist gekennzeichnet durch Gefäßkontraktion, Aktivierung der Gerinnungskaskade und komplexe immunologische Abläufe. Es kommt in der Regel zur Ausbildung eines Fibrinnetzes, welches die Wunde verschließt und nach außen schützt. Durch die Freisetzung vasoaktiver Substanzen (z.B. Histamin und Serotonin) kann eine lokale Entzündungsreaktion hervorgerufen werden. Die umliegenden Gefäße können sich erweitern und durch eine gesteigerte Kapillarpermeabilität können Leukozyten zum Entzündungsort wandern. Diese können Mikroorganismen und Gewebsnekrosen beseitigen. Dadurch kann eine Reinigung der Wunde erfolgen.

Die darauffolgende Proliferations- oder Granulationsphase beginnt üblicherweise etwa am zweiten Tag nach der Wundentstehung und kann z.B. bis zu 14 Tage andauern. Es erfolgt der Aufbau von neuem Gewebe mit Gefäßeinsprossung und Defektauffüllung durch Granulationsgewebe. Dies ist die Grundvoraussetzung für die spätere Epithelisierung. Fibroblasten aus dem umliegenden Gewebe können in das Fibrinnetz migrieren und es als provisorische Matrix nutzen. Es beginnt der Aufbau von Kollagenfasern. Durch das Enzym Plasmin kann das Fibringerüst mittels Fibrinolyse abgebaut werden. Die verschlossenen Gefäße können rekanalisiert werden.

Mit der Differenzierungs- oder Umbauphase beginnt, etwa zwischen dem sechsten und zehnten Tag, üblicherweise die Ausreifung der kollagenen Fasern. Die Wunde kontrahiert sich durch die Umwandlung von Fibroblasten in Fibrozyten sowie Myofibroblasten. Dadurch schrumpft das Narbengewebe und es führt zu einer Verkleinerung der Wunde. Die Epithelisierung vom Wundrand her bringt die Wundheilung zum Abschluss.

In einer bevorzugten Ausführungsform der vorliegenden Erfindung ist die Behandlung der Wunden, bevorzugt der sekundär heilenden Wunden, eine phasengerechte Wundbehandlung. Unter einer phasengerechten Wundtherapie wird im Rahmen dieser Anmeldung verstanden, dass die Wundtherapie auf die spezifischen Bedürfnisse der Wunde in den einzelnen Phasen eingeht.

So kann die phasengerechte Behandlung gezielt in einer oder mehreren Phasen der Wundheilung erfolgen. (Im Gegensatz dazu erfolgt bei herkömmlichen Wundbehandlungen ein und dieselbe Behandlung über alle Phasen).

Der Begriff Hydrogel bezeichnet dabei im Rahmen der Erfindung ein feindisperses System aus mindestens einer festen und einer flüssigen Phase. Diese feste Phase bildet dabei ein schwammartiges, dreidimensionales Netzwerk, dessen Poren durch eine Flüssigkeit (Lyogel) beziehungsweise auch ein Gas (Xerogel) ausgefüllt sind. Beide Phasen durchdringen sich dabei bevorzugt vollständig. Durch Wasseraufnahme kann das dreidimensionale Netzwerk durch Quellen sein Volumen vergrößern, ohne dabei den strukturellen Zusammenhalt zu verlieren. Ein Hydrogel kann bevorzugt aus einem synthetischen oder natürlichen Material, bevorzugt aus einem hydrophilen synthetischen Material, aufgebaut sein.

Der Begriff Hydrogel wird im Folgenden auch synonym als Hydrogelzusammensetzung oder Hydrogelmatrix bezeichnet.

Die vorliegende Erfindung weist dabei als feste Phase ein Polymer enthaltend Polyurethan- und Polyharnstoffgruppen. Als flüssige Phase dient dabei eine Mischung aus Wasser und mehrwertigem Alkohol, ausgenommen Propylenglykol.

Im Folgenden werden Prozentangaben, die sich auf die Konzentration von Inhaltsstoffen beziehen, so verstanden, dass sie den Anteil der genannten Ausgangsstoffe in der Gesamtreaktionsmischung aus Präpolymeren, Wasser, mehrwertigem Alkohol und ggf. Salz bezeichnen.

Als wasserhaltige Hydrogele können im Zusammenhang mit der vorliegenden Erfindung insbesondere Hydrogele verwendet werden, die eine zusammenhängende, diskrete Schicht bilden und unter einem Druck, der bei bestimmungsgemäßer Anwendung des Hydrogels auftritt, kein Wasser abgeben.

Die vorliegende Erfindung betrifft wasserhaltige Hydrogele zur Behandlung von Wunden erhältlich durch Umsetzung eines a) Amin-terminierten Präpolymers enthaltend Polyalkylenoxideinheiten mit einem b) Isocyanat-terminierten Präpolymer enthaltend Polyalkylenoxideinheiten, wobei die Umsetzung in Gegenwart eines mehrwertigen Alkohols, ausgenommen Propylenglykol, und in Gegenwart von Wasser erfolgt und wobei bezogen auf die Gesamtmasse aller Reaktanden die Summe der Massen aus Amin-terminiertem Präpolymer und Isocyanat-terminiertem Präpolymer 10-30 Gew.-% der Gesamtmasse aller Reaktanden beträgt und die Masse des mehrwertigen Alkohols, ausgenommen Propylenglykol, 5-35 Gew.-% der Gesamtmasse aller Reaktanden beträgt und die Masse des eingesetzten Wasser mindestens 40 Gew.-% der Gesamtmasse aller Reaktanden beträgt, wobei das molare Verhältnis reaktiver Isocyanatendgruppen zu reaktiven Aminendgruppen 1,0 bis 2,0, bevorzugt 1,0 bis 1,8, besonders bevorzugt 1,0 bis 1,6, ganz besonders bevorzugt 1,0 bis 1,5, am meisten bevorzugt 1,2 bis 1,3 dadurch gekennzeichnet, dass es sich bei dem mehrwertigen Alkohol um Glycerol handelt.

In einer bevorzugten Ausführungsform betrifft die Erfindung ein wasserhaltiges Hydrogel zur Behandlung von Wunden erhältlich durch Umsetzung eines a) Amin-terminierten Präpolymers enthaltend Polyalkylenoxideinheiten mit einem b) Isocyanat-terminierten Präpolymer enthaltend Polyalkylenoxideinheiten, wobei die Umsetzung in Gegenwart eines mehrwertigen Alkohols, ausgenommen Propylenglykol, und in Gegenwart von Wasser erfolgt und wobei bezogen auf die Gesamtmasse aller Reaktanden die Summe der Massen aus Amin-terminiertem Präpolymer und Isocyanat-terminiertem Präpolymer 15-25 Gew.-% der Gesamtmasse aller Reaktanden beträgt und die Masse des mehrwertigen Alkohols, ausgenommen Propylenglykol, 5-35 Gew.-% der Gesamtmasse aller Reaktanden beträgt und die Masse des eingesetzten Wasser mindestens 40 Gew.-% der Gesamtmasse aller Reaktanden beträgt, wobei das molare Verhältnis reaktiver Isocyanatendgruppen zu reaktiven Aminendgruppen 1,0 bis 2,0, bevorzugt 1,0 bis 1,8, besonders bevorzugt 1,0 bis 1,6, ganz besonders bevorzugt 1,0 bis 1,5, am meisten bevorzugt 1,2 bis 1,3 dadurch gekennzeichnet, dass es sich bei dem mehrwertigen Alkohol um Glycerol handelt.

Insbesondere ist das Hydrogel dabei erhältlich durch Umsetzung eines Amin-terminierten Präpolymers enthaltend Polyethylenoxid- und/oder Polypropylenoxideinheiten mit einem mindestens dreiarmig verzweigten Isocyanat-terminierten Präpolymer enthaltend Polyethylenoxid- und/oder Polypropylenoxideinheiten in Gegenwart eines mehrwertigen Alkohols, ausgenommen Propylenglykol, wobei das molare Verhältnis reaktiver Isocyanatendgruppen zu reaktiven Aminendgruppen 1,0 bis 2,0, bevorzugt 1,0 bis 1,8, besonders bevorzugt 1,0 bis 1,6, ganz besonders bevorzugt 1,0 bis 1,5, am meisten bevorzugt 1,2 bis 1,3 beträgt.

Insbesondere ist das Hydrogel dabei erhältlich durch Umsetzung eines Amin-terminierten Präpolymers enthaltend Polyethylenoxid- und Polypropylenoxideinheiten mit einem mindestens dreiarmig verzweigten Isocyanat-terminierten Präpolymer enthaltend Polyethylenoxid- und Polypropylenoxideinheiten in Gegenwart eines mehrwertigen Alkohols, ausgenommen Propylenglykol, wobei das Verhältnis reaktiver Isocyanatendgruppen zu reaktiven Aminendgruppen 1,0 bis 2,0, bevorzugt 1,0 bis 1,8, besonders bevorzugt 1,0 bis 1,6, ganz besonders bevorzugt 1,0 bis 1,5, am meisten bevorzugt 1,2 bis 1,3 beträgt und wobei das Gewichtsverhältnis von Polyethylenoxid- zu Polypropylenoxideinheiten sowohl im Amin-terminierten Präpolymer als auch im Isocyanat-terminierten Präpolymer 3 : 1 bis 7 : 1 beträgt.

Ein typisches Amin-terminiertes Präpolymer ist beispielsweise ein Triblock-Polymer aus Propylenglykol-, Ethylenglykol- und wieder Propylenglykoleinheiten, wobei das Polymer endständig jeweils mit 2-Aminopropylgruppen aminfunktionalisiert ist. Es weist einen Gehalt reaktiver Aminendgruppen von 0,9554 mmol/g bei einem Molekulargewicht von durchschnittlich etwa 2000g/mol und einer Dispersität von 1,08, gemessen durch Gelpermeationschromatographie, auf und ein molares Verhältnis von Ethyleneinheiten zu Propyleneinheiten von 3:1 bis 7:1, bevorzugt 39:6. Ein derartiges Amin-terminiertes Präpolymer ist beispielsweise kommerziell erhältlich als Jeffamin® ED-2003, Huntsman; Everberg, Belgien.

Ein typisches Isocyanat-terminiertes Präpolymer mit aliphatischen Diisocyanat-Gruppen ist beispielsweise ein dreiarmiges Copolymer aus Propylenglykol- und Polyethylenglykoleinheiten, welches endständig jeweils mit einem Molekül Isophorondiisocyanat umgesetzt wurde. Es weist einen Gehalt reaktiver Isocyanatendgruppen (NCO-Gruppen) von 3,0% bis 3,4%, bevorzugt 3,2% auf und ein molares Verhältnis von Ethylenoxideinheiten zu Propylenoxideinheiten von 3 : 1 bis 4:1. ein derartiges isocyanat-terminiertes Präpolymer mit aliphatischen Diisocyanat-Gruppen ist beispielsweise kommerziell erhältlich als Aquapol® PL-13000-3 (Carpenter; Richmond, USA).

Besonders bevorzugt ist ein Hydrogel, das erhältlich ist durch Umsetzung des besagten Amin-terminierten Präpolymers Jeffamin® ED-2003 mit dem besagten Isocyanat-terminierten Aquapol® PL-13000-3 in Gegenwart eines mehrwertigen Alkohols, ausgenommen Propylenglykol, wobei das Massenverhältnis von Aquapol zu Jeffamin zwischen 1,0 und 2,5, bevorzugt zwischen 1,1 und 1,7 liegt. Diese Gele zeigen eine ausreichende Gelfestigkeit für die Verwendung in Wundauflagen, während bei einem niedrigeren Verhältnis die erhaltenen Produkte viskose Flüssigkeiten darstellen und bei einem höheren Verhältnis die erhaltenen Produkte starre Gelkörper darstellen.

Diese Hydrogele sind besonders gut geeignet Wasser einzulagern und dieses Wasser an eine Wunde abzugeben.

Bei den genannten Hydrogelen wird die feste Phase nicht allein durch ein Polymer gebildet, das aus der Reaktion zwischen einem Amin-terminierten Präpolymer und einem Isocyanat-terminierten Präpolymer entsteht. An der Reaktion ist ebenfalls ein mehrwertiger Alkohol, ausgenommen Propylenglykol, beteiligt, dessen freie Hydroxygruppen mit Isocyanatgruppen reagieren können. Die Komponente des mehrwertigen Alkohols trägt dabei zu einer zusätzlichen Vernetzung bei, die insbesondere bei mehrwertigen Alkoholen mit mehr als zwei Hydroxygruppen zu einer dreidimensionalen Vernetzung der Präpolymere führen. In der Reaktion zwischen einem mehrwertigen Alkohol und einer Isocyanatgruppe entsteht ein Carbamidsäureester, der auch als Urethan bezeichnet wird. Diese Reaktion kann durch Säuren oder Basen als Katalysator beschleunigt werden und unter Zufuhr von thermischer Energie rückgängig gemacht werden. Wird die Reaktionstemperatur konstant zwischen 5°C und 30°C, bevorzugt zwischen 5°C und 20°C, gehalten, kann diese Reaktion in einem ausreichenden Verhältnis erfolgen, so dass Hydrogele mit kovalent eingebundenen mehrwertigen Alkoholen erhalten werden, die vorteilhafte Eigenschaften aufweisen.

Weiterhinenthalten erfindungsgemäße Hydrogele Glycerol. Dieser Alkohol ist hervorragend als Feuchtigkeitsspender geeignet und stellt somit für die die Wunde umgebende Haut eine pflegende Komponente dar. Hydrogele, die diesen Alkohol als Partner in den oben beschriebenen Reaktionen enthalten, weisen ein hohes Absorptionsvermögen für Wundexsudat und einen verringerten Feuchtigkeitsverlust auf. Sie weisen eine Adhäsionskraft auf, die einen atraumatischen Verbandwechsel erlaubt. Aufgrund ihrer geringen Zytotoxizität sind sie sehr gut verträglich für Wundgewebe. Außerdem sind derartige Gele in der Lage, für die Wundheilung notwendige Wachstumsfaktoren des Wundexsudats aufzukonzentrieren und so die Wundheilung zu beschleunigen. Dies gilt in besonderem Maße für Glycerol-haltige Hydrogele.

In einer bevorzugten Ausführungsform wird als mehrwertiger Alkohol Glycerol in einer Konzentration von 10-25 Gew.-% verwendet. Derartige Hydrogele weisen besonders vorteilhafte Eigenschaften hinsichtlich der Zellkompatibilität, des Flüssigkeitsverlusts und des Haftvermögens auf.

In einer besonders bevorzugten Ausführungsform wird als mehrwertiger Alkohol Glycerol in einer Konzentration von 15-25 Gew.-% verwendet. Derartige Hydrogele weisen außerdem ein besonders hohes Absorptionsvermögen auf.

Ein erfindungsgemäßes Hydrogel enthält mindestens 40 Gew.-% und ganz besonders bevorzugt mindestens 50 Gew.-% Wasser, wobei das Hydrogel weiterhin bevorzugt höchstens 90 Gew.-% und weiterhin bevorzugt höchsten 80 Gew.-% Wasser umfasst. Somit kann ein Hydrogel zur Wundbehandlung bereitgestellt werden, das eine für eine natürliche Wundheilung ausreichende Menge Feuchtigkeit zur Verfügung stellt.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Bevorzugt umfasst die Hydrogelmatrix Natriumchlorid, Kaliumchlorid, Magnesiumchlorid, Kalziumchlorid oder Mischungen hiervon. Besonders bevorzugt ist Natriumchlorid. Diese Salze simulieren in besonders guter Weise das Elektrolyt-Gemisch in einem von einer Wunde abgegebenen Wundserum. Damit stellt eine diese Salze umfassende Hydrogelmatrix einer Wunde ein besonders wundheilungsförderndes Klima zur Verfügung.

Hierbei kann vorgesehen sein, dass die Hydrogelmatrix 0 bis 5 Gew.-% mindestens ein Salz umfasst. Insbesondere umfasst die Hydrogelmatrix 0,1 bis 3 Gew.-% eines Salzes und ganz besonders bevorzugt 0,5 bis 1,5 Gew.-% eines Salzes.

Erfindungsgemäße Hydrogele sind für die Behandlung von Wunden geeignet. Sie können als halbfeste, plastisch verformbare Massen mittels geeigneter Applikationsvorrichtungen auf eine Wundoberfläche verteilt werden. Geeignete Applikationsvorrichtungen sind dabei beispielsweisen Tuben oder angepasste Spritzen.

Erfindungsgemäße Hydrogele sind bevorzugt Bestandteil einer Wundauflage. Unter einer Wundauflage wird im Rahmen der vorliegenden Erfindung ein Produkt verstanden, das auf eine Wunde aufgebracht wird in gebrauchsfertiger Form zur Verfügung gestellt wird. Geeignete Wundauflagen weisen dabei wenigstens eine Schicht aus einem Trägermaterial und eine Schicht umfassend ein erfindungsgemäßes Hydrogel auf.

Als Trägerschicht können insbesondere Polymerfilme oder Polymerschäume eingesetzt werden, vorzugsweise Filme oder Schäume, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässiger und wasserdampfdurchlässiger Polyurethanfilm oder ein wasserundurchlässiger und wasserdampfdurchlässiger Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 $\mu$m bis 50 $\mu$m, insbesondere 20 $\mu$m bis 40 $\mu$m und ganz besonders bevorzugt von 25 $\mu$m bis 30 $\mu$m aufweisen. Die Wasserdampfdurchlässigkeit des Polymerfilms der Wundauflage weist vorzugsweise mindestens 750 g/m$^2$/24h, insbesondere mindestens 1000 g/m$^2$/24h und ganz besonders bevorzugt mindestens 2000 g/m$^2$/24h auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass das Wundsystem an seinem bestimmungsgemäßen Ort appliziert und fixiert werden kann. Darüber hinaus ist sichergestellt, dass keine Flüssigkeit zwischen der Folie und der die zu behandelnden Fläche umgebende Haut austreten kann. Als besonders bevorzugt sind solche Klebstoffe zu betrachten, die in einem dünnen Auftrag von 20 g/m$^2$ bis 35 g/m$^2$ zusammen mit dem Film eine Wasserdampfdurchlässigkeit von mindestens 800 g/m$^2$/24h und vorzugsweise von mindestens 1000 g/m$^2$/24h (gemessen nach DIN EN 13726) aufweisen.

In einer bevorzugten Ausführungsform umfasst die absorbierende Schicht einen hydrophilen Polyurethanschaum sowie das Hydrogel. Beispielsweise kann die Oberfläche eines hydrophilen Polyurethanschaums mit dem Hydrogel imprägniert bzw. beschichtet oder von diesem ganz oder teilweise durchsetzt sein.

In einer alternativen Ausführungsform kann die Hydrogelzusammensetzung auch benachbart oder räumlich getrennt von der absorbierenden Schicht vorliegen. Beispielsweise kann die Hydrogelzusammensetzung, beispielsweise umfassend ein Polyurethan-Polyharnstoff-Copolymer auf eine Oberfläche einer Schicht aus einem Polyurethanschaum beschichtet werden, so dass eine die Hydrogelzusammensetzung umfassende Hydrogelschicht in direktem Kontakt auf einer Schicht aus Polyurethanschaum aufliegt. Alternativ können die Hydrogelschicht und die absorbierende Schicht auch durch eine Abstandsschicht voneinander getrennt sein. Beispielsweise kann die Abstandschicht eine Hydrogelmatrix, ein Polymerfilm, eine Hydrokolloidmatrix, ein Polymernetz, ein Textilgewebe, ein Adhäsiv und/oder ein Polymernetz umfassen.

Ferner kann die mehrschichtige Wundauflage auch weitere Schichten neben der absorbierenden Schicht und der Trägerschicht umfassen, wie beispielsweise eine Wundkontaktschicht, eine oder mehrere Barriereschichten und/oder eine oder mehrere Verteilerschichten.

Bevorzugte Wundauflagen umfassen eine Trägerschicht, eine Hydrogelschicht gemäß der vorliegenden Erfindung und fakultativ eine zwischen der Hydrogelschicht und der Trägerschicht angeordnete absorbierende Schicht. Die absorbierende Schicht kann bevorzugt ein Fasermaterial, besonders bevorzugt einen hydrophilen Polyurethanschaum umfassen. Die Hydrogelschicht kann kontinuierlich oder diskontinuierlich sein. Sie kann beispielsweise vollflächig auf die Trägerschicht aufgebracht sein oder Kanäle, Löcher oder anders geformte Öffnungen aufweisen. Bei einer diskontinuierlichen Hydrogelschicht kann eine Vielzahl diskreter Hydrogelelemente auf der Trägerschicht und/oder auf der absorbierenden Schicht aufgebracht sein, die die Form von Kreisen, Quadraten oder anderen regelmäßigen oder unregelmäßigen Vielecken aufweisen können.

Mögliche Anordnungen der verschiedenen Schichten in erfindungsgemäßen mehrschichtigen Wundauflagen sind beispielsweise in der WO 2010/000450 beschrieben.

Die Wundauflagen weisen ferner einen hohen Komfort für den Patienten auf, indem sie einfach anzuwenden, hautfreundlich, weich, dünn, hautanpassend und analgetisch (über einen Hydrogel-Kühlungseffekt) sind und können somit auch über einen langen Zeitraum angewendet werden, üblicherweise 3 bis 5 Tagen, bevor ein Wechsel der Wundauflage erfolgt. Das Trägermaterial kann vollflächig oder partiell, kontinuierlich oder diskontinuierlich mit einem Klebstoff beschichtet sein.

In einer erfindungsgemäßen Wundauflage kann ein erfindungsgemäßes Hydrogel direkt auf einer Trägerschicht aufgetragen sein. Es kann auch mit Hilfe eines Klebstoffes zum besseren Zusammenhalt aufgebracht sein. Zwischen der Trägerschicht und der Hydrogelschicht können weitere Schichten angeordnet sein. Es hat sich als vorteilhaft herausgestellt, wenn eine Wundauflage als zusätzliche Schicht eine Schicht aufweist, die in der Lage ist, Flüssigkeit aufzunehmen, zu speichern und/oder innerhalb der Schicht zu verteilen oder zu weiteren Schichten zu transferieren. Geeignete Schichten, die in der Lage sind Flüssigkeiten aufzunehmen, sind Vliesmaterialien aus natürlichen oder synthetischen Fasern oder Mischungen davon, offenporige Schaummaterialien oder Materialien, die eine hydrophobe Matrix enthalten, in der Flüssigkeit aufnehmende Partikel enthalten sind. Bevorzugt ist ein offenporiges Schaummaterial aus Polyurethan. Ein erfindungsgemäßes Wundversorgungsprodukt umfasst bevorzugt einen hydrophilen Polyurethanschaum. Die Verwendung eines hydrophilen Polyurethanschaums ist für eine schnelle Wundheilung vorteilhaft, weil solche Schäume eine hohe Absorptionskapazität aufweisen und daher vorzugsweise in der Reinigungsphase der Wundheilung bei starker Exsudation eingesetzt werden. Ein weiterer Vorteil von Polyurethanschäumen besteht darin, dass nur geringe Scherkräfte auf eine zu behandelnde Wunde ausgeübt werden und die Wunde somit gut abgepolstert wird.

Im Zusammenhang mit der vorliegenden Erfindung wird unter einem hydrophilen Polyurethanschaum ein Polyurethanschaum verstanden, der eine Flüssigkeit in seine Polyurethanmatrix und in seine Poren aufnehmen und speichern, somit absorbieren kann, und zumindest einen Teil der aufgenommenen Flüssigkeit wieder abgeben kann. Als hydrophile Polymerschäume sind hierbei insbesondere offenporige, hydrophile Polyurethanschäume geeignet. Demgemäß umfasst eine besonders bevorzugte Wundauflage eine Schicht, die einen offenporigen, hydrophilen Polyurethanschaum umfasst. Erfindungsgemäß sollen vorzugsweise Polyurethanschäume eingesetzt werden, die eine hohe Absorptionskapazität für Flüssigkeiten von mehr als 2,5 g, bevorzugt mehr als 10 g, noch mehr bevorzugt mehr als 16 g isotonischer Salzlösung pro Gramm Schaumpolymer aufweisen. Die Absorptionsfähigkeit wird nach DIN EN 13726-1:2002 (3 min Messung) bestimmt. Ein derartiger Schaum kann Keime und Zelltrümmer absorbieren und sicher einschließen, dabei aber trotzdem weich, geschmeidig und mit einer guten Polsterwirkung auf der Wunde aufliegen.

Vorzugsweise weist der hydrophile Polyurethanschaum eine durchschnittliche Porengröße von weniger als 1000 $\mu$m, insbesondere 100 bis 1000 $\mu$m, bevorzugt 100 bis 500 $\mu$m und ganz besonders bevorzugt 100 bis 300 $\mu$m auf. Die bevorzugte Methode zur Bestimmung der Porengröße ist die Messung des Durchmessers einer Vielzahl von Poren auf einer Schnittebene, die parallel zur Wundkontaktseite der Schaumschicht bzw. des Wundversorgungsprodukts orientiert ist. Die Messung der Porengröße kann durch Betrachten der Poren in einem Licht- oder Elektronenmikroskop und Vergleich des Porendurchmessers mit einem geeigneten Maßstab erfolgen. Der Schaum kann eine homogene Porengröße oder einen Gradienten der Porengröße über die Dicke der Schaumschicht aufweisen. Bei Verwendung eines Schaums, welcher einen Gradienten der Porengröße ausweist, wird durch eine von der Wundkontaktschicht ausgehende Verringerung der Porengröße von größeren Poren an der Wundkontaktseite (mittlere Porengröße z.B. 200-300 $\mu$m) bis hin zu kleineren Poren an der im Gebrauch von der Wunde abgewandten Seite des Schaums (mittlere Porengröße z.B. 100-200 $\mu$m) eine effiziente Ableitung von Wundexsudat gewährleistet. Eine effiziente Ableitung von Wundexsudat ergibt sich, weil ein Kapillareffekt für eine besonders gute Absorption von Flüssigkeiten erzeugt werden kann. Gleichzeitig kann der Schaum eine ausreichende Menge an Feuchtigkeit für eine Wunde bereitstellen. Ein Schaum mit einem Gradienten der Porengröße über die Dicke des Schaums und einer Porengröße von weniger als 1000 $\mu$m kommt beispielsweise bei dem Produkt Permafoam der Paul Hartmann AG zum Einsatz. Darüber hinaus ist es besonders vorteilhaft, wenn die Wundauflage außerdem eine wasserdampf-durchlässige Polyurethan-Deckschicht aufweist. Weiterhin ist es vorteilhaft, wenn die wasserdampfdurchlässige Polyurethan-Deckschicht eine Wasserdampfdurchlässigkeit ("upright", gemessen nach DIN EN 13726-2 bei einer Temperatur von 37°C) von mehr als 600 g/m$^2$ in 24 h aufweist.

Denkbar und vorteilhaft ist es darüber hinaus, wenn die Wundauflage wundseitig ein netzförmiges Hydrogel aufweist. Weiterhin ist es vorteilhaft, wenn die Wundauflage wundabseitig eine Polyurethan-Deckschicht umfasst.

Des Weiteren ist es von Vorteil, wenn der Schaum eine Dichte von 70 bis 110 kg/m3 aufweist. Gemäß einer weiteren Ausführung der Erfindung kann ein hydrophober PU-Schaum mit einer Dichte von 10 bis 50 kg/m3 verwendet werden. Derartige Schäume werden insbesondere bei Wundauflagen, welche für eine Unterdruck-Therapie von Wunden vorgesehen sind, eingesetzt. In einer weiteren Ausführung der Erfindung wäre es weiterhin denkbar und vorteilhaft, Silikonschäume mit einer Dichte von bis zu 300 kg/m3 einzusetzen.

Polyurethanschaumstoffe sind üblicherweise erhältlich durch Umsetzung einer härtbaren Mischung, umfassend die Komponenten Polyisocyanat und gegenüber Isocyanat reaktiver Verbindungen, insbesondere Polyol, sowie Katalysatoren, Treibmitteln und gegebenenfalls Zusatzstoffen. Als Isocyanate können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat, hier insbesondere 4,4'-Diphenylmethandiisocyanat, Mischungen aus monomeren Diphenylmethandiisocyanaten und höherkernigen Homologen des Diphenylmethandiisocyanats, Tetramethylendiisocyanat, Hexamethylendiisocyanat, Toluylendiisocyanat oder Mischungen daraus. Als gegenüber Isocyanaten reaktive Verbindungen werden üblicherweise Polyole wie Polyetherole und/oder Polyesterole verwendet.

Als besonders vorteilhafte Ausführungen haben sich weiterhin Schaumwundauflagen gezeigt, die einen Polyurethanschaum umfassen, dessen Schichtdicke 0,1 cm bis 1,8 cm, bevorzugt von 0,3 cm bis 1,5 cm und ganz besonders bevorzugt von 0,5 cm bis 1,0 cm, aufweist. Die Schichtdicke kann an jeder Stelle der Wundkontaktschicht gleich sein oder in verschiedenen Bereichen der Wundkontaktschicht verschiedene Werte annehmen. Insbesondere ist auch vorgesehen, dass die absorbierende Schicht oder der Polyurethanschaum abgeflachte Ränder aufweist.

Bevorzugt weist das Wundversorgungsprodukt eine im Wesentlichen quadratische Grundform auf. Besonders bevorzugt ist dabei ein Größenbereich von 8 cm x 8 cm bis zu 20 cm x 20 cm. Die Dicke des Wundversorgungsprodukts beträgt bevorzugt weniger als 2 cm, wobei die Schaumschicht bevorzugt eine Dicke zwischen 0,1 cm und 1,8 cm aufweist.

Als Wundkontaktschicht kann gemäß der vorliegenden Erfindung ein zusätzliches Material Verwendung finden. Hierbei steht eine Wundkontaktschicht bei Verwendung der erfindungsgemäßen Wundauflage in direktem Kontakt mit der Wunde. Die Wundkontaktschicht kann einzig und allein dazu dienen, den Schaum von der zu behandelnden Wunde zu beabstanden. Die zusätzliche Schicht hat den Vorteil, bei einem Verbandswechsel eine besonders gewebeschonende Ablösung des Wundversorgungsprodukts zu gewährleisten. Die Wundkontaktschicht kann weitere Funktionen in Bezug auf die zu behandelnde Wunde ausüben. Beispielsweise kann die Wundkontaktschicht die Wunde mit Feuchtigkeit versorgen, wundrandpflegende Eigenschaften aufweisen, Hautirritationen vermindern oder antiadherent wirken.

Eine erfindungsgemäße Wundauflage kann eine Wundkontaktschicht aufweisen, wobei die Wundkontaktschicht ein Hydrogel, einen Polymerfilm, eine Hydrokolloidmatrix, ein Polymernetz, ein Nonwoven und/oder ein Adhäsiv umfasst. Der Begriff Hydrogel bzw. Gel bezeichnet dabei im Rahmen der Erfindung ein feindisperses System aus mindestens einer festen und einer flüssigen Phase. Diese feste Phase bildet dabei ein schwammartiges, dreidimensionales Netzwerk, dessen Poren durch eine Flüssigkeit (Lyogel) bzw. auch ein Gas (Xerogel) ausgefüllt sind. Beide Phasen durchdringen sich dabei vollständig.

Damit soll im Zusammenhang mit der vorliegenden Erfindung unter einem hydrophilen Polymerschaum mit einem Wasseranteil von mindestens 10 Gew.-% oder einem hydrophilen Polyurethanschaum mit einem Wasseranteil von mindestens 10 Gew.-% ein solcher Polymerschaum oder Polyurethanschaum verstanden sein, der mindestens 10 Gew.-% Wasser umfasst, wobei das Wasser von dem Polymerschaum oder dem Polyurethanschaum freigesetzt werden kann. Im Unterschied hierzu ist nicht der Anteil an Wasser zu verstehen, der eventuell zur Bildung beispielsweise bei der Polymerisation der Ausgangsprodukte des Polymerschaums oder des Polyurethanschaums verwendet wird. Dieses Wasser wird kovalent gebunden und steht nicht der Wundbehandlung zur Verfügung. Darüber hinaus soll auch nicht ein Wasseranteil verstanden sein, der produktionsbedingt bei der Herstellung des Schaums verwendet wird. Dieser Wasseranteil wird nach oder während der Bildung des Polymerschaums dem Schaum meist durch Trocknen, beispielsweise durch Trocknen in einem Ofen, entzogen und steht somit auch nicht der Wundbehandlung zur Verfügung. Damit weist eine erfindungsgemäße Wundauflage einen Polymerschaum oder einen Polyurethanschaum auf, der einen Wasseranteil umfasst, der deutlich über einem eventuell durch die Herstellung nach Trocknung bedingten Restgehalt an Wasser übersteigt.

Weiterhin bevorzugt umfasst eine erfindungsgemäße Wundauflage einen hydrophilen Polyurethanschaum, der einen Retentionswert R von mindestens 20 % aufweist. Hierbei ist weiterhin bevorzugt vorgesehen, dass der hydrophile Polyurethanschaum einen Retentionswert R von mindestens 30 %, insbesondere von mindestens 40 %, insbesondere mindestens 40 % und ganz besonders bevorzugt von mindestens 50 % aufweist. Unabhängig hiervon kann weiterhin bevorzugt vorgesehen sein, dass die Wundauflage einen hydrophilen Polyurethanschaum aufweist, der einen Retentionswert R von höchstens 90 %, insbesondere von höchstens 80 % und ganz besonders von höchstens 70 % aufweist. Der Retentionswert R wird dabei gemäß einer hierin beschriebenen Methode bestimmt.

Ganz besonders bevorzugt umfasst eine erfindungsgemäße Wundauflage einen hydrophilen Polyurethanschaum, der einen Wasseranteil von mindestens 10 Gew.-% umfasst, wobei der Wasseranteil dem Retentionswert R des Polyurethanschaums entspricht.

Das Hydrogel kann auf unterschiedliche Arten zur Wundbehandlung eingesetzt werden. Das Gel kann zuerst auf die Wunde aufgetragen und dann mit einer Wundauflage abgedeckt werden. Eine andere Möglichkeit, das Gel zur Wundbehandlung einzusetzen, besteht darin, eine Wundauflage zu verwenden, die das Hydrogel in einer Wundkontaktschicht bereithält. Auf diese Weise werden die den pH-Wert im sauren Bereich stabilisierenden Eigenschaften des Gels in der Wunde bereitgestellt.

Als besonders vorteilhafte Ausführungen haben sich weiterhin Wundauflagen gezeigt, die eine Hydrogelmatrix umfassen, deren Schichtdicke 0,1 bis 5,0 mm aufweist. Insbesondere weist damit eine erfindungsgemäße Wundauflage eine Wundkontaktschicht mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken zeigen einerseits keine Wundverklebung und andererseits die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Weiterhin bevorzugt kann die Hydrogelmatrix Kanäle umfassen, insbesondere konische Kanäle, zum Durchtritt von Flüssigkeiten von der ersten zur zweiten Seite. Hierdurch kann insbesondere ein verbesserter Durchtritt für Wundexsudat bereitgestellt werden. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sind. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Dabei ist ganz besonders bevorzugt vorgesehen, dass die erste Seite Öffnungen aufweist, die im Vergleich zu der auf der zweiten Seite befindliche Öffnung größer ist.

Gemäß einer Weiterbildung der Erfindung kann auch vorgesehen sein, dass die Wundkontaktschicht oder die Hydrogelmatrix Öffnungen aufweist, die einen Durchmesser von 0,5 bis 5 mm aufweisen. Insbesondere weist die Wundkontaktschicht oder die Hydrogelmatrix Öffnungen auf, die einen Durchmesser von 1 bis 3 mm aufweisen. Ganz besonders bevorzugt weist die Wundkontaktschicht oder die Hydrogelmatrix auf der wundzugewandten ersten Seite Öffnungen auf, die einen Durchmesser von 1 bis 3 mm aufweisen, wobei die zweite Seite der Wundkontaktschicht oder der Hydrogelmatrix in direktem Kontakt mit dem Polyurethanschaum steht.

Es kann jedoch auch vorgesehen sein, dass zwischen der absorbierenden Schicht und der Wundkontaktschicht eine Übergangsschicht angeordnet ist. In dieser Ausführungsform weist eine erfindungsgemäße Wundauflage zwischen der Hydrogelmatrix und Polyurethanschaum eine Schicht auf, die beide Materialen umfasst. Diese Übergangsschicht kann ebenso wie die Wundkontaktschicht Kanäle, Öffnungen oder Löcher aufweisen. Falls die Übergangsschicht Kanäle, Öffnungen oder Löcher aufweist, sind gemäß einer weiter bevorzugten Ausführungsform diese Kanäle, Öffnungen oder Löcher mit Polyurethanschaum ausgefüllt. Weiterhin bevorzugt sind diese Kanäle, Öffnungen oder Löcher kongruent zu den Kanälen, Öffnungen oder Löchern der Wundkontaktschicht. Durch die Anordnung einer solchen Übergangsschicht kann eine Wundauflage bereitgestellt werden, die ein Laminat aus einem Polyurethanschaum und einer Hydrogelmatrix umfasst, das einen besonders festen Zusammenhalt zwischen der absorbierenden Schicht und der Wundkontaktschicht aufweist.

Die erfindungsgemäßen Hydrogele sind für die Behandlung von Wunden geeignet. Die vorliegende Erfindung umfasst daher auch erfindungsgemäße Hydrogele zur Behandlung von Wunden. Insbesondere umfasst die vorliegende Erfindung Hydrogele zur Behandlung chronischer Wunden wie Dekubitus, Druck-Ulzera, Druckgeschwüre, Ulcus cruris venosum, venöse Ulzera, Ulcus cruris arteriosum, arterielle Ulzera, Wunden infolge von diabetischem Fußsyndrom, neuropathische Ulzera, aber auch Wunden in Folge von Autoimmunerkrankungen oder von Tumoren (exulzierende Tumore) oder von Strahlenschäden bei der Tumortherapie.

Erfindungsgemäße Hydrogele bzw. Wundauflagen, die diese enthalten, sind zur phasengerechten Wundtherapie geeignet, insbesondere zur Therapie von Wunden in der Granulationsphase und/oder der Epithelisierungsphase.

**Beschreibung der Figuren**

Figur 1: Eine erste erfindungsgemäße Wundauflage
Figur 2: Eine zweite erfindungsgemäße Wundauflage im Querschnitt
Figur 3: Eine dritte erfindungsgemäße Wundauflage im Querschnitt
Figur 3a: Ein Teilausschnitt der dritten erfindungsgemäße Wundauflage im Querschnitt

Figur 4: Eine vierte erfindungsgemäße Wundauflage im Querschnitt

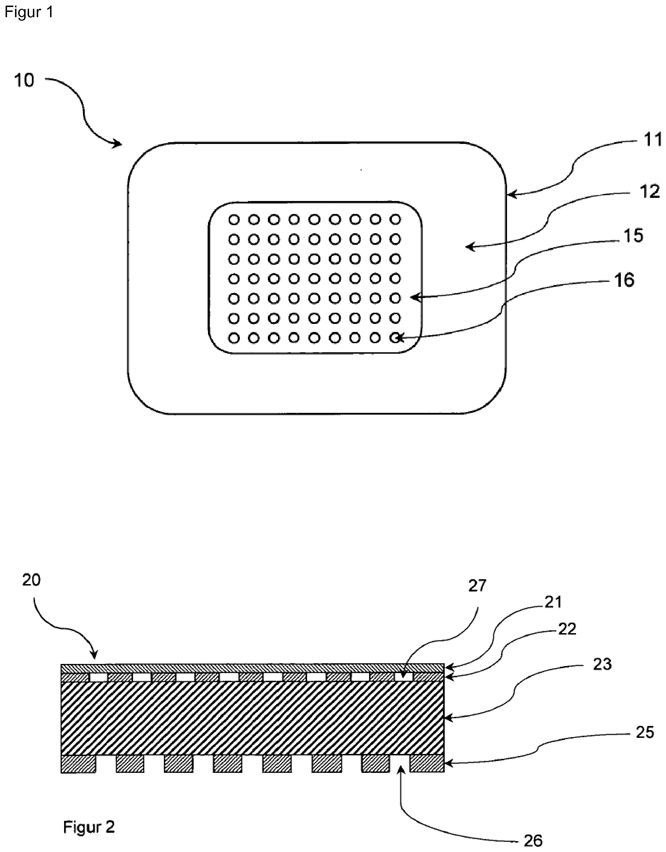

Mit Figur 1 ist eine erste erfindungsgemäße Wundauflage (10) mit Sicht auf die Wundkontaktschicht gezeigt. Die Wundauflage (10) ist als so genannter Inselverband gefertigt und besteht aus einer Trägerschicht (11) aus einem wasserundurchlässigen und wasserdampfdurchlässigen Polyurethanfilm, die vollflächig mit einem Acrylatkleber (12) beschichtet ist. Im Zentrum der Trägerschicht ist mittels des Acrylatklebers (12) eine absorbierende hydrophile Polyurethanschaumschicht aufgebracht (hier nicht dargestellt), auf die ein erfindungsgemäßes Hydrogel (15) als Wundkontaktschicht aufgebracht ist. Die hydrophile Polyurethanschaumschicht umfasst einen Wasseranteil von 40 Gew.-% Wasser. Somit umfassen 100 g eines in diesem Beispiel verwendeten Polyurethanschaums 40 g Wasser und 60 g Polyurethanmatrix. Die Hydrogel-Wundkontaktschicht (15) ist adhäsiv mit der absorbierenden Polyurethanschaumschicht (hier nicht dargestellt) verbunden. In die Hydrogel-Wundkontaktschicht sind eine Vielzahl an kreisförmigen Löchern (16) eingebracht, um den Durchfluss von Wundexsudat von der Wunde in die absorbierende Schicht zu ermöglichen. Durch die Hydrogel-Wundkontaktschicht wird ein Einwachsen von neu gebildeten Zellen in die Poren des Polyurethanschaums verhindert.

Mit Figur 2 ist eine weitere Ausführungsform einer erfindungsgemäßen Wundauflage gezeigt. Die Wundauflage (20) umfasst eine mit einer absorbierenden Schicht (23) kongruente Trägerschicht (21) aus einem wasserundurchlässigen und wasserdampfdurchlässigen Polyurethanschaum, die mittels einer diskontinuierlichen adhäsiven Schicht (22) aus einem Acrylatkleber mit der absorbierenden Schicht (23) verbunden ist. Durch den diskontinuierlichen Auftrag bleiben Bereiche (27) der absorbierenden Schicht und der Trägerschicht unverbunden. Die Wundauflage umfasst eine absorbierende Schicht (23) mit einer Schichtdicke von 4 mm und eine Trägerschicht (21) mit einer Schichtdicke von 1,5 mm. Die absorbierende Schicht (23) wird aus einem offenporigen hydrophilen Polyurethanschaum gebildet, der eine Porengröße von durchschnittlich 220 $\mu$m aufweist. Der Polyurethanschaum umfasst dabei einen Wasseranteil von 70 Gew.-%. Auf die erste Seite des Polyurethanschaums ist ein erfindungsgemäßes Hydrogel als Wundkontaktschicht (25) aufgebracht. Das Hydrogel ist mit einem Flächengenwicht von 75 g/m$^2$ nicht kontinuierlich auf dem Polyurethanschaum aufgebracht, so dass in der Hydrogel-Wundkontaktschicht (25) kreisförmige Löcher (26) zum verbesserten Durchtritt von Wundexsudat vorgesehen sind. Der Polyurethanschaum umfasst eine erste Seite mit einer Fläche von 25 cm$^2$, wobei die Löcher (26) insgesamt eine Fläche von 5 cm$^2$ einnehmen.

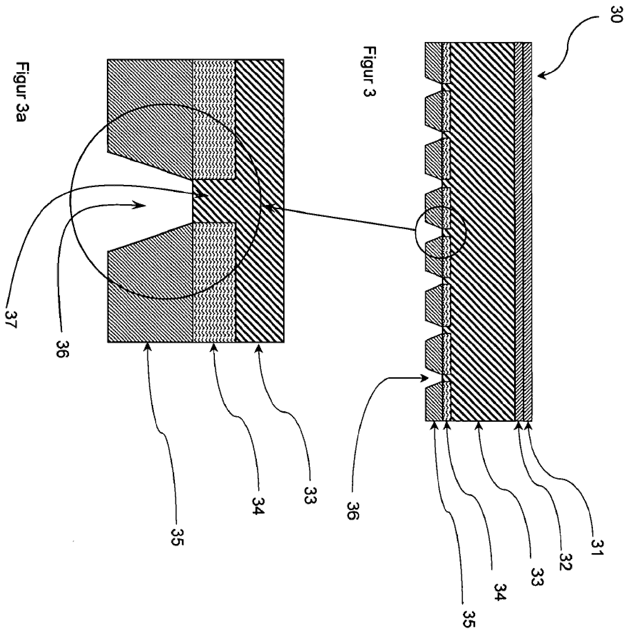

Mit Figur 3 ist eine dritte Ausführung einer erfindungsgemäßen Wundauflage gezeigt. Die Wundauflage (30) umfasst eine Trägerschicht (31) aus einem wasserundurchlässigen und wasserdampfdurchlässigen Polyurethanfilm, eine absorbierende Schicht (33) aus einem offenporigen hydrophilen Polyurethanschaum mit einem Wasseranteil von 52,8 Gew.-% (bezogen auf den Polyurethanschaum) und einer Wundkontaktschicht (35) aus einem erfindungsgemäßen Hydrogel mit einem Wasseranteil von ca. 57,9 Gew.-% auf (bezogen auf das Hydrogel). Die Trägerschicht (31) ist vollflächig mittels einem auf den Polymerfilm aufgetragenem Acrylatkleber (32) auf den hydrophilen Polymerschaum auflaminiert. Auf der im Anwendungsfall zur Wunde weisenden ersten Seite der absorbierenden Schicht ist ein erfindungsgemäßes wasserhaltiges Hydrogel (35) aufgebracht, das ein Polyurethan-Polyharnstoff-Copolymer umfasst. Die Hydrogel-Wundkontaktschicht ist mit konischen, im Querschnitt (parallel zur Wunde) kreisförmigen Kanälen (36) ausgestattet, so dass ein verbesserter Wundexsudatfluss von der Wunde in den absorbierenden hydrophilen Schaum erfolgen kann (vgl. Figur 3a). Bei der Herstellung der Wundauflage ist das noch viskose Hydrogel geringfügig in den Polyurethanschaum eingedrungen, so dass zwischen der Hydrogel-Wundkontaktschicht und dem hydrophilen Polyurethanschaum eine Übergangsschicht (34) ausgebildet ist, die aus dem Hydrogel und dem hydrophilen Polyurethanschaum besteht. Die Übergangsschicht ihrerseits weist Kanäle (37) auf, die nur mit Polyurethanschaum gefüllt sind und die kongruent zu den Kanälen in der Hydrogel-Wundkontaktschicht angeordnet sind.

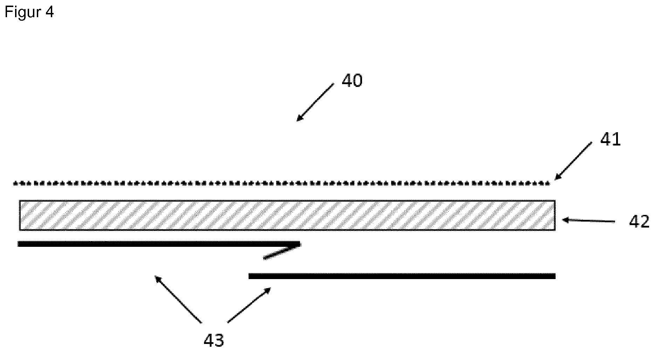

Mit Figur 4 ist eine vierte Ausführungsform einer erfindungsgemäßen Wundauflage gezeigt. Die Wundauflage (40) umfasst eine Trägerschicht (41) aus einem wasserundurchlässigen und wasserdampfdurchlässigen Polyurethanfilm, eine Schicht (42) aus erfindungsgemäßem wasserhaltigen Hydrogel und eine zweiteilige Abdeckschicht (43) aus silikonisiertem Papier. Die Hydrogelschicht weist eine Dicke von 3mm auf.

**Beispiele:**

Beispiele 1-13: Herstellung der Gele
(Beispiele 7-13 sind nicht erfindungsgemäß)

Entsprechend der folgenden Tabelle 1 werden Mischungen aus Alkohol, demineralisertem Wasser und Natriumchlorid hergestellt.

Tabelle 1: Mischungen aus Alkohol, Wasser und Natriumchlorid

| Beispiel Nr. | Bezeichnung Gel | Alkohol | Demin. Wasser | NaCl |
|---|---|---|---|---|
| 1 | Glycerol 5% | 70,4 g Glycerol | 916,0 g | 13,6 g |
| 2 | Glycerol 10% | 140,8 g Glycerol | 845,6 g | 13,6 g |
| 3 | Glycerol 15% | 211,3 g Glycerol | 775,1 g | 13,6 g |
| 4 | Glycerol 20% | 281,7 g Glycerol | 704,7 g | 13,6 g |
| 5 | Glycerol 25% | 352,1 g Glycerol | 634,3 g | 13,6 g |
| 6 | Glycerol 30% | 422,5 g Glycerol | 563,9 g | 13,6 g |
| 7 | Ethylenglykol 20% | 281,7 g Ethylenglykol | 704,7 g | 13,6 g |
| 8 | Sorbitol 20% | 281,7 g Sorbitol | 704,7 g | 13,6 g |
| 9 | Saccharose 20% | 281,7 g Saccharose | 704,7 g | 13,6 g |
| 10 | PEG300 20% | 281,7 g PEG 300 | 704,7 g | 13,6 g |
| 11 | PEG2000 20% | 281,7 g PEG 2000 | 704,7 g | 13,6 g |
| 12 | $H_2O$ | - | 986,4 g | 13,6 g |
| 13 | Propylen glykol | 281,7 g Propylenglykol | 704,7 g | 13,6 g |

In einem zweiten Schritt werden 3,465g Jeffamin bei 50°C geschmolzen und mit 3,135g demineralisiertem Wasser vermischt. Diese Menge Jeffamin enthält 3,31mmol reaktive Aminendgruppen. Die erhaltene Mischung wird mit 28,4g einer entsprechend der Tabelle hergestellten Alkohol-Mischung und 5,0 g Aquapol unter heftigem Rühren und Eiswasserkühlung vermischt. Diese Menge Aquapol enthält 3,84mmol reaktive Isocyanatendgruppen. Das molare Verhältnis reaktiver Isocyanatendgruppen zu reaktiven Amingruppen beträgt dabei jeweils 1,16. Die erhaltene Mischung wird ausgegossen und zu einem Gel mit einer Dicke von 3mm ausgestrichen.

Die Massen der in der Reaktion zur Herstellung der Gele eingesetzten Bestandteile weisen die in der Tabelle 2 angegebenen Verhältnisse bezogen auf die Gesamtmasse der eingesetzten Reaktanden auf:

Tabelle 2: Massenverhältnisse der eingesetzten Reaktanden

| Beispiel Nr. | Jeffamin ED-2003 | Aquapol PL-13000-3 | Alkohol | Demin. Wasser | NaCl |
|---|---|---|---|---|---|
| 1 | 8,7% | 12,5% | 5,0% | 72,9% | 1,0% |
| 2 | 8,7% | 12,5% | 10,0% | 67,9% | 1,0% |
| 3 | 8,7% | 12,5% | 15,0% | 62,9% | 1,0% |
| 4 | 8,7% | 12,5% | 20,0% | 57,9% | 1,0% |
| 5 | 8,7% | 12,5% | 25,0% | 52,9% | 1,0% |
| 6 | 8,7% | 12,5% | 30,0% | 47,9% | 1,0% |
| 7 | 8,7% | 12,5% | 20,0% | 57,9% | 1,0% |
| 8 | 8,7% | 12,5% | 20,0% | 57,9% | 1,0% |
| 9 | 8,7% | 12,5% | 20,0% | 57,9% | 1,0% |
| 10 | 8,7% | 12,5% | 20,0% | 57,9% | 1,0% |
| 11 | 8,7% | 12,5% | 20,0% | 57,9% | 1,0% |
| 12 | 8,7% | 12,5% | 0,0% | 77,9% | 1,0% |
| 13 | 8,7% | 12,5% | 20,0% | 57,9% | 1,0% |

Bei Beispiel Nr. 12 handelt es sich um ein Vergleichsbeispiel ohne mehrwertigen Alkohol.
Bei Beispiel Nr. 13 handelt es sich um ein Vergleichsbeispiel gemäß WO2010/000451.

Beispiel 14: Messung des Feuchtigkeitsverlusts:

Als Feuchtigkeitsverlust wird der Verlust an Gewicht über einen bestimmten Zeitraum bei einer definierten Temperatur beschrieben. Der Feuchtigkeitsverlust berechnet sich nach folgender Gleichung und wird in der Einheit g/g angegeben:

$$Feuchtigkeitsverlust = \frac{Endgewicht}{Anfangsgewicht}$$

Für die Gele der Ausführungsbeispiele wurden die in der Tabelle 3 angegebenen Feuchtigkeitsverluste ermittelt:

Tabelle 3: Feuchtigkeitsverlust in g/g Gel

| Beispiel Nr. | Probe | Feuchtigkeitsverlust [g/g] | | | | |
|---|---|---|---|---|---|---|
| | | 2h | 4h | 6h | 8h | 24h |
| 1 | Glycerol 5% | 0,857 | 0,763 | 0,716 | 0,682 | 0,583 |
| 2 | Glycerol 10% | 0,861 | 0,779 | 0,724 | 0,702 | 0,641 |
| 3 | Glycerol 15% | 0,865 | 0,791 | 0,747 | 0,734 | 0,726 |
| 4 | Glycerol 20% | 0,884 | 0,827 | 0,785 | 0,756 | 0,688 |
| 5 | Glycerol 25% | 0,927 | 0,874 | 0,840 | 0,824 | 0,802 |
| 6 | Glycerol 30% | 0,951 | 0,914 | 0,894 | 0,883 | 0,864 |
| 7 | Ethylenglykol 20% | 0,943 | 0,910 | 0,887 | 0,874 | 0,819 |
| 8 | Sorbitol 20% | 0,804 | 0,698 | 0,637 | 0,596 | 0,510 |
| 9 | Saccharose 20% | 0,795 | 0,672 | 0,631 | 0,611 | 0,582 |
| 10 | PEG300 20% | 0,828 | 0,708 | 0,663 | 0,646 | 0,638 |
| 11 | PEG2000 % | 0,806 | 0,710 | 0,645 | 0,604 | 0,516 |
| 12 | H2O | 0,781 | 0,641 | 0,560 | 0,505 | 0,299 |
| 13 | Propylenglykol 20% | 0,832 | 0,720 | 0,655 | 0,617 | 0,529 |

Beispiel 15: Messung der Absorptionskapazität

Zur Messung der Absorptionskapazität werden Gelproben mit Durchmesser $\varnothing$ 5 cm ausgestanzt. Anschließend werden sie in ein Becherglas mit V = 300 ml deionisiertem Wasser gegeben. Daraufhin werden sie in bestimmten Zeitabständen erneut gewogen. Die Berechnung der Absorptionskapazität erfolgt nach folgender Gleichung und wird in der Einheit g/g angegeben:

$$Absorptionskapazität = \frac{Endgewicht - Anfangsgewicht}{Anfangsgewicht}$$

Für die Gele der Ausführungsbeispiele wurden die in der Tabelle 4 angegebenen Absorptionskapazitäten ermittelt:

Tabelle 4: Absorptionskapazität in g/g Gel

| Beispiel Nr. | Probe | Absorptionskapazität [g/g] | | | | |
|---|---|---|---|---|---|---|
| | | 2h | 4h | 6h | 8h | 24h |
| 1 | Glycerol 5% | 1,195 | 1,574 | 1,844 | 2,027 | 2,511 |
| 2 | Glycerol 10% | 1,209 | 1,581 | 1,859 | 2,061 | 2,707 |

(fortgesetzt)

| Beispiel Nr. | Probe | Absorptionskapazität [g/g] | | | | |
|---|---|---|---|---|---|---|
| | | 2h | 4h | 6h | 8h | 24h |
| 3 | Glycerol 15% | 1,635 | 2,237 | 2,654 | 3,043 | 3,894 |
| 4 | Glycerol 20% | 1,647 | 2,171 | 2,514 | 2,815 | 3,706 |
| 5 | Glycerol 25% | 1,743 | 2,337 | 2,806 | 3,096 | 4,111 |
| 6 | Glycerol 30% | 1,845 | 2,463 | 2,855 | 3,157 | 4,183 |
| 7 | Ethylenglykol 20% | 2,200 | 3,026 | 3,663 | 3,945 | 5,331 |
| 8 | Sorbitol 20% | 1,997 | 2,734 | 3,377 | 3,965 | 5,994 |
| 9 | Saccharose 20% | 2,704 | 3,498 | 4,124 | 4,587 | 5,877 |
| 10 | PEG300 20% | 2,759 | 3,641 | 4,245 | 4,785 | 6,820 |
| 11 | PEG2000 20% | 1,527 | 2,276 | 2,597 | 2,819 | 3,044 |
| 12 | H2O | 1,230 | 1,703 | 2,022 | 2,141 | 2,670 |
| 13 | Propylenglykol 20% | 1,694 | 2,313 | 2,655 | 2,958 | 3,836 |

Beispiel 16: Messung der Adhäsionskraft

Der Begriff Adhäsionskraft beschreibt die Fähigkeit eines Klebstoffs an einer Oberfläche zu haften. Sie entspricht der Kraft, die notwendig ist, um einen mit der Geloberfläche in Kontakt geratenen Körper von dieser Oberfläche zu lösen, und wird mit Hilfe einer statischen Material-Prüfmaschine Zwick 010 ermittelt. Die Prüfungen werden bei einer Standardtemperatur von T = 23 °C und einer relativen Luftfeuchtigkeit von 50 %rh durchgeführt. Die Gele müssen für 24 Stunden vor der Prüfung unter den Prüfbedingungen konditioniert werden. Für jede Messung werden jeweils drei Proben mit einer Größe von jeweils 5 cm x 5 cm aus den Gelen ausgestanzt. Die Proben werden mit der wundabgewandten Seite mit einem doppelseitigen Klebeband auf einem horizontal beweglichen Schlitten befestigt. Die Anfahrgeschwindigkeit des Schlittens beträgt 100 mm/min, die Kontaktzeit mit der Geloberfläche 2 s, die Abzugsgeschwindigkeit des Schlittens 400 mm/min. Der Prüfkörper (Gewicht = 0,245 N) bewegt sich abwärts, bis dieser mit der Oberfläche des Gels in Kontakt tritt, wo er für eine Zeit von t = 2 s verweilt. Nach dieser Kontaktzeit bewegt sich der Prüfkörper nach oben und misst die Kraft, die zum Lösen des Körpers von der Geloberfläche notwendig ist.

Beispiel 17: Test auf Zellkompatibilität

Die Tests auf Zellkompatibilität wurden gemäß DIN EN ISO 10993-5 und den Verfahrensanweisungen der Abteilung für Funktionswerkstoffe der Medizin und der Zahnheilkunde: BioLab 973302, 042901, 964702 und 964805 durchgeführt und umfassen Messungen des Zellwachstums, der Stoffwechselaktivität und des Proteingehalts.

Die Hydrogele wurden steril in Petrischalen geliefert. Zur Prüfung wurden je 0,1 g/ml Kulturmedium der Proben eingewogen.

Die Zellaktivität, die Zellzahl sowie die Proteinkonzentration wurden pro Probe dreimal in je vier Parallelansätzen untersucht. Die Eluationszeit betrug 48 h, die Inkubation der Zellen mit den Eluaten ebenso 48 h.

Als Zelllinie wurden L 929 CC1 Mausfibroblasten, American Type Culture Collection, Rockeville Maryland USA verwendet.

Als Kulturmedium wurde DMEM (Dulbecco's mod. Eagle Medium) nach VA BioLab 042901 zur Vorkultur und Eluation verwendet.

Als Negativkontrolle wurde Polystyrol der Firma Nunc GmbH & Co KG, Wiesbaden verwendet. Als Positivkontrolle wurden Vekoplan KT PVC Platten der Firma König GmbH, Wendelstein verwendet.

Pro Probe wurden drei Eluate aus je einem Hydrogel getestet, die an unterschiedlichen Versuchstagen angesetzt wurden. Hierzu wurden die Hydrogele in den Petrischalen mit einem sterilen Skalpell mittig halbiert und in ein steriles 50 ml Reaktionsgefäß überführt. Pro 0,1 g Probe wurde 1 ml Eluationsmedium zu den Hydrogelen zugegeben und diese dann für 48 h bei 37 °C und 5 % $CO_2$ im Inkubator eluiert. Um vorhandene Schwebstoffe aus den Eluaten zu entfernen, wurden die Proben nach der Inkubation für 5 min bei 4000 rpm zetrifugiert und durch einen Filter (Porengröße 0,2 $\mu$m) filtriert.

Die Zellen wurden in der Konzentration 50.000 Zellen/ml ausgesät, die Vorkultivierung erfolgte bei 37 °C und

5 % $CO_2$ für 24 h. Anschließend wurde das bei der Aussaat zugegebene DMEM Medium abgezogen und die Zellen mit je 1 ml Eluat in der Konzentration 100 % bedeckt. Als Negativkontrolle wurde DMEM Medium 48 h in einem 50 ml Falconröhrchen wie die Proben inkubiert, als Positivkontrolle diente das Eluat der Plastic Discs in der Konzentration 100 %. Nach 48 stündiger Inkubation wurden die Zellaktivität, die Zellzahl und der Gesamtproteingehalt bestimmt.

*Zellwachstum*

Die Zellzählung erfolgte nach enzymatischer Ablösung der Zellen mittels Accutase mit Hilfe des Zellzählers.

*Vitalitätstest über Stoffwechselaktivität*

Die Prüfung der Vitalität erfolgt mit Tetrazoliumsalz, WST 1, Fa. Roche Diagnostics GmbH Mannheim, nach Angabe des Herstellers. WST 1 wird durch die Succinatdehydrogenase (Enzym des Citronensäurezyklus) in den Mitochondrien der stoffwechselaktiven Zellen zum farbigen Formazan umgesetzt und photometrisch vermessen. Die Absorptionswerte (OD), bei 450 nm und 620 nm bestimmt, korrelieren mit der Atmungsaktivität der kultivierten Zellen.

*Proteingehalt*

Die Prüfung des Proteingehaltes erfolgt mit dem DC Protein Assay, Fa. BIO-RAD GmbH München, nach Angabe des Herstellers. Die Proteinbestimmung nach Lowry basiert auf der Reduktion von Cu(ll) zu Cu(l) durch die aromatischen Tyrosin- Tryptophanreste von Proteinen. Der Kupfer-Protein-Komplex reduziert in einem weiteren Schritt ein Phosphormolybdänsäure-Phosphorwolframat-Reagenz zu Molybdän bzw. Wolframblau. Die Extinktion dieser intensiven blauen Färbung wird photometrisch bei 750 nm gemessen. Durch das Mitführen einer Standardreihe kann die Proteinkonzentration bestimmt werden.

*Akzeptanz und Auswertung*

Die Einteilung der Wertungsbereiche für Akzeptanz und Auswertung erfolgte in Anlehnung an DIN EN ISO 7405 und dem Begriff Inhibitionsdosis (ID 50: Dosis bei welcher 50% der Zellen im Wachstum gehemmt sind) (Literatur: Allgemeine Pharmakologie und Toxikologie, Henschler, Hrsg.: Forth Wolfgang; Spektrum akad. Verl. Heidelberg; 7. Aufl. 1996). Als starke Wachstumshemmung wird ein Zellwachstum von 0-29 %, als mäßige Hemmung ein Zellwachstum von 30-59 % und als schwache Hemmung ein Zellwachstum von 60-79 % im Vergleich zur Kontrolle charakterisiert. Zellwachstumsraten zwischen 80 und 100 % indizieren ein ungehemmtes Zellwachstum.

Als stark verminderte Stoffwechselaktivität wird eine Zellaktivität von 0-29 %, als mäßig verminderte Stoffwechselaktivität eine Zellaktivität von 30-59 % und als schwach verminderte Stoffwechselaktivität eine Zellaktivität von 60-79 % im Vergleich zur Kontrolle charakterisiert. Zellaktivitätsraten zwischen 80 und 100% indizieren eine nicht verminderte Stoffwechselaktivität.

Als stark reduzierter Proteingehalt wird eine Proteinkonzentration von 0-34%, als mäßig reduzierter Proteingehalt eine Proteinkonzentration von 35-69 % im Vergleich zur Kontrolle charakterisiert. Proteinkonzentrationen zwischen 70 und 100 % indizieren einen nicht reduzierten Proteingehalt.

Für die Gele der Ausführungsbeispiele wurden die in der Tabelle 5 angegebenen Zellkompatibilitäten ermittelt:

Tabelle 5: Zellkompatibilität

| Beispiel Nr. | Probe | Hemmung des Zellwachstums | Verminderung der Stoffwechselaktivität | Reduktion des Proteingehalts |
|---|---|---|---|---|
| 1 | Glycerol 5% | Schwach | Mäßig | Mäßig |
| 2 | Glycerol 10% | schwach | Schwach | Keine |
| 3 | Glycerol 15% | Schwach | Schwach | Keine |
| 4 | Glycerol 20% | Schwach | Schwach | Keine |
| 5 | Glycerol 25% | Schwach | Schwach | Keine |
| 6 | Glycerol 30% | mäßig | schwach | keine |
| 7 | Ethylenglykol 20% | Mäßig | mäßig | Mäßig |

**EP 3 558 400 B1**

(fortgesetzt)

| Beispiel Nr. | Probe | Hemmung des Zellwachstums | Verminderung der Stoffwechselaktivität | Reduktion des Proteingehalts |
|---|---|---|---|---|
| 8 | Sorbitol 20% | Mäßig | stark | Mäßig |
| 9 | Saccharose 20% | Schwach | mäßig | Keine |
| 10 | PEG300 20% | stark | Stark | Mäßig |
| 11 | PEG2000 20% | mäßig | mäßig | Mäßig |
| 12 | H2O | schwach | schwach | Mäßig |
| 13 | Propylenglykol 20% | Stark | Stark | Mäßig |

**Patentansprüche**

1. Wasserhaltiges Hydrogel zur Behandlung von Wunden erhältlich durch Umsetzung eines a) Amin-terminierten Präpolymers enthaltend Polyalkylenoxideinheiten mit einem b) Isocyanat-terminierten Präpolymer enthaltend Polyalkylenoxideinheiten, wobei die Umsetzung in Gegenwart eines mehrwertigen Alkohols, ausgenommen Propylenglykol, und in Gegenwart von Wasser erfolgt und wobei bezogen auf die Gesamtmasse aller Reaktanden

    die Summe der Massen aus Amin-terminiertem Präpolymer und Isocyanat-terminiertem Präpolymer 10-30 Gew.-% der Gesamtmasse aller Reaktanden beträgt und
    die Masse des mehrwertigen Alkohols, ausgenommen Propylenglykol, 5-35 Gew.-% der Gesamtmasse aller Reaktanden beträgt und
    die Masse des eingesetzten Wasser mindestens 40 Gew.-% der Gesamtmasse aller Reaktanden beträgt, wobei das molare Verhältnis reaktiver Isocyanatendgruppen zu reaktiven Aminendgruppen 1,0 bis 2,0, bevorzugt 1,0 bis 1,5 beträgt, **dadurch gekennzeichnet, dass** es sich bei dem mehrwertigen Alkohol um Glycerol handelt.

2. Wasserhaltiges Hydrogel gemäß Anspruch 1, **dadurch gekennzeichnet dass** das Hydrogel erhältlich ist durch Umsetzung eines a) Amin-terminierten Präpolymers enthaltend Polyethylenoxid- und/oder Polypropylenoxideinheiten mit einem b) mindestens dreiarmig verzweigten Isocyanat-terminierten Präpolymer enthaltend Polyethylenoxid- und/oder Polypropylenoxideinheiten.

3. Wasserhaltiges Hydrogel gemäß einem der vorangehenden Ansprüche, **dadurch gekennzeichnet, dass** das Gewichtsverhältnis von Polyethylenoxid- zu Polypropylenoxideinheiten sowohl im Amin-terminierten Präpolymer als auch im Isocyanat-terminierten Präpolymer 3 : 1 bis 7 : 1 beträgt.

4. Wasserhaltiges Hydrogel nach einem der vorangehenden Ansprüche, **dadurch gekennzeichnet, dass** Glycerol in einer Menge von 10-25 Gew.-% eingesetzt wird.

5. Wasserhaltiges Hydrogel nach einem der vorangehenden Ansprüche, **dadurch gekennzeichnet, dass** Glycerol in einer Menge von 15-25 Gew.-% eingesetzt wird.

6. Wasserhaltiges Hydrogel gemäß einem der vorangehenden Ansprüche, **dadurch gekennzeichnet, dass** die Hydrogelmatrix zusätzlich 0,5-1,5 Gew.-% eines Salzes, ausgewählt aus der Gruppe Natriumchlorid, Kaliumchlorid, Magnesiumchlorid und/oder Calciumchlorid umfasst.

7. Wundauflage umfassend eine Rückschicht und eine Schicht umfassend eine wasserhaltige Hydrogelmatrix gemäß einem der vorangegangenen Ansprüche.

8. Wundauflage gemäß Anspruch 7, **dadurch gekennzeichnet, dass** die Wundauflage zusätzlich eine absorbierende Schicht aufweist, die zwischen der Rückschicht und der Schicht umfassend eine wasserhaltige Hydrogelmatrix gemäß einem der Ansprüche 1-6 angeordnet ist.

**9.** Wundauflage gemäß einem der Ansprüche 7 oder 8, **dadurch gekennzeichnet, dass** die Schicht umfassend eine wasserhaltige Hydrogelmatrix gemäß einem der Ansprüche 1-6 kontinuierlich ist.

**10.** Wundauflage gemäß einem der Ansprüche 7 bis 9, **dadurch gekennzeichnet dass** die Schicht umfassend eine wasserhaltige Hydrogelmatrix gemäß einem der Ansprüche 1- 6 Löcher, Öffnungen oder Kanäle aufweist.

**11.** Wasserhaltiges Hydrogel gemäß einem der Ansprüche 1 bis 6 zur Anwendung in der Behandlung chronischer Wunden.

**12.** Wasserhaltiges Hydrogel gemäß einem der Ansprüche 1 bis 6 zur Aufkonzentrierung von wundheilungsfördernden Wachstumsfaktoren.

**13.** Wasserhaltiges Hydrogel gemäß einem der Ansprüche 1 bis 6 zur Behandlung von Wunden in der Granulations- und/oder Epithelisierungsphase.

**Claims**

**1.** Aqueous hydrogel for the treatment of wounds obtainable by reacting an a) amine-terminated prepolymer containing polyalkylene oxide units with an b) isocyanate-terminated prepolymer containing polyalkylene oxide units, the reaction taking place in the presence of a polyhydric alcohol, except propylene glycol, and in the presence of water and, based on the total mass of all reactants,

   the sum total of the masses of amine-terminated prepolymer and isocyanate-terminated prepolymer being 10-30% by weight of the total mass of all reactants and
   the mass of the polyhydric alcohol, except propylene glycol, being 5-35% by weight of the total mass of all reactants and
   the mass of the water used being at least 40% by weight of the total mass of all reactants,
   the molar ratio of reactive isocyanate end groups to reactive amine end groups being 1.0 to 2.0, preferably 1.0 to 1.5, **characterized in that** the polyhydric alcohol is glycerol.

**2.** Aqueous hydrogel according to Claim 1, **characterized in that** the hydrogel is obtainable by reacting an a) amine-terminated prepolymer containing polyethylene oxide units and/or polypropylene oxide units with an b) at least three-armedly branched isocyanate-terminated prepolymer containing polyethylene oxide units and/or polypropylene oxide units.

**3.** Aqueous hydrogel according to either of the preceding claims, **characterized in that** the weight ratio of polyethylene oxide units to polypropylene oxide units both in the amine-terminated prepolymer and in the isocyanate-terminated prepolymer is 3:1 to 7:1.

**4.** Aqueous hydrogel according to any of the preceding claims, **characterized in that** glycerol is used in an amount of 10-25% by weight.

**5.** Aqueous hydrogel according to any of the preceding claims, **characterized in that** glycerol is used in an amount of 15-25% by weight.

**6.** Aqueous hydrogel according to any of the preceding claims, **characterized in that** the hydrogel matrix additionally comprises 0.5-1.5% by weight of a salt selected from the group consisting of sodium chloride, potassium chloride, magnesium chloride and/or calcium chloride.

**7.** Wound dressing comprising a backing layer and a layer comprising an aqueous hydrogel matrix according to any of the preceding claims.

**8.** Wound dressing according to Claim 7, **characterized in that** the wound dressing additionally comprises an absorbent layer which is arranged between the backing layer and the layer comprising an aqueous hydrogel matrix according to any of Claims 1-6.

**9.** Wound dressing according to either of Claims 7 and 8, **characterized in that** the layer comprising an aqueous hydrogel matrix according to any of Claims 1-6 is continuous.

10. Wound dressing according to any of Claims 7 to 9, **characterized in that** the layer comprising an aqueous hydrogel matrix according to any of Claims 1-6 comprises holes, openings or channels.

11. Aqueous hydrogel according to any of Claims 1 to 6 for use in the treatment of chronic wounds.

12. Aqueous hydrogel according to any of Claims 1 to 6 for the concentration of wound healing-promoting growth factors.

13. Aqueous hydrogel according to any of Claims 1 to 6 for the treatment of wounds in the granulation and/or epithelialization phase.


## Revendications

1. Hydrogel aqueux destiné au traitement de plaies, pouvant être obtenu par mise en réaction d'un a) prépolymère à terminaison amino, contenant des motifs polyoxyalkylène, avec un b) prépolymère à terminaison isocyanate, contenant des motifs polyoxyalkylène, dans lequel la réaction s'effectue en présence d'un alcool polyhydrique, à l'exclusion du propylèneglycol, et en présence d'eau et dans lequel, par rapport à la masse totale de tous les partenaires réactionnels,

    la somme des masses de prépolymère à terminaison amino et de prépolymère à terminaison isocyanate représente 10-30 % en poids de la masse totale de tous les partenaires réactionnels et
    la masse de l'alcool polyhydrique, à l'exclusion du propylèneglycol, représente 5-35 % en poids de la masse totale de tous les partenaires réactionnels et
    la masse de l'eau utilisée représente au moins 40 % en poids de la masse totale de tous les partenaires réactionnels,
    dans lequel le rapport molaire des groupes terminaux isocyanate réactifs aux groupes terminaux amino réactifs vaut de 1,0 à 2,0, de préférence de 1,0 à 1,5, **caractérisé en ce que** pour ce qui est de l'alcool polyhydrique il s'agit du glycérol.

2. Hydrogel aqueux selon la revendication 1, **caractérisé en ce que** l'hydrogel peut être obtenu par mise en réaction d'un a) prépolymère à terminaison amino, contenant des motifs polyoxyéthylène et/ou polyoxypropylène, avec un b) prépolymère à terminaison isocyanate ramifié en au moins trois branches, contenant des motifs polyoxyéthylène et/ou polyoxypropylène.

3. Hydrogel aqueux selon l'une quelconque des revendications précédentes, **caractérisé en ce que** le rapport pondéral des motifs polyoxyéthylène aux motifs polyoxypropylène aussi bien dans le prépolymère à terminaison amino que dans le prépolymère à terminaison isocyanate vaut de 3 : 1 à 7 : 1.

4. Hydrogel aqueux selon l'une quelconque des revendications précédentes, **caractérisé en ce que** le glycérol est utilisé en une quantité de 10-25 % en poids.

5. Hydrogel aqueux selon l'une quelconque des revendications précédentes, **caractérisé en ce que** le glycérol est utilisé en une quantité de 15-25 % en poids.

6. Hydrogel aqueux selon l'une quelconque des revendications précédentes, **caractérisé en ce que** la matrice d'hydrogel comprend en outre 0,5-1,5 % en poids d'un sel choisi dans le groupe constitué par le chlorure de sodium, le chlorure de potassium, le chlorure de magnésium et/ou le chlorure de calcium.

7. Pansement comprenant une couche dorsale et une couche comprenant une matrice d'hydrogel aqueux selon l'une quelconque des revendications précédentes.

8. Pansement selon la revendication 7, **caractérisé en ce que** le pansement comporte en outre une couche absorbante, qui est disposée entre la couche dorsale et la couche comprenant une matrice d'hydrogel aqueux selon l'une quelconque des revendications 1-6.

9. Pansement selon l'une quelconque des revendications 7 et 8, **caractérisé en ce que** la couche comprenant une matrice d'hydrogel aqueux selon l'une quelconque des revendications 1-6 est continue.

10. Pansement selon l'une quelconque des revendications 7 à 9, **caractérisé en ce que** la couche comprenant une matrice d'hydrogel aqueux selon l'une quelconque des revendications 1-6 comporte des trous, des ouvertures ou des canaux.

11. Hydrogel aqueux selon l'une quelconque des revendications 1 à 6, destiné à l'utilisation dans le traitement de plaies chroniques.

12. Hydrogel aqueux selon l'une quelconque des revendications 1 à 6, destiné à la concentration de facteurs de croissance favorisant la cicatrisation.

13. Hydrogel aqueux selon l'une quelconque des revendications 1 à 6, destiné au traitement de plaies dans la phase de granulation et/ou d'épithélialisation.

Figur 1

Figur 2

30

31
32
33
34
35

Figur 3

36

33

34

35

Figur 3a

36

37

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 630629 A
- WO 2010000450 A
- WO 2010000451 A
- EP 2338528 A
- EP 2338529 A

**In der Beschreibung aufgeführte Nicht-Patentliteratur**

- Allgemeine Pharmakologie und Toxikologie. Spektrum akad. Verl, 1996